# EUROPEAN PATENT APPLICATION

(11) **EP 1 849 483 A2**
(43) Date of publication of application: **31.10.2007**
(21) Application number: 07006448.0
(22) Date of filing: 28.03.2007

(54) **Bioabsorbable tube and production method thereof**

(30) Priority: 30.03.2006 JP 2006094551; 08.09.2006 JP 2006244528
(71) Applicant: GC Corporation, Tokyo 174-8585 (JP)
(72) Inventor: Yamamoto, Katsushi, Tokyo 174-8585 (JP); Yamanaka, Katsuyuki, Tokyo 174-8585 (JP); Suda, Youko, Tokyo 174-8585 (JP); Kaneko, Tadashi, Tokyo 174-8585 (JP)
(74) Representative: Müller-Boré & Partner Patentanwälte

(57) **Abstract**

An objective of the present invention is to provide a bioabsorbable tube for regenerating nerve tissue and a production method thereof, where the tube can be produced easier than a conventional bioabsorbable tube for regenerating nerve tissue with a low cost, and therefore, various kinds of the tubes having different thicknesses, inner diameters and outer shapes can be easily formed. A bioabsorbable tube is produced by: drying a solvent of a solution dissolved with an bioabsorbable polymer so as to form a sheet-like bioabsorbable polymer material; cylindrically rolling the sheet-like bioabsorbable polymer material so as to form an overlapped sheet part; and heat sealing or solvent meld sealing at least a portion around an end edge on the outer peripheral side of the overlapped sheet part . Or a bioabsorbable tube having a two layers structure is produced by: freeze-drying a solvent of a solution dissolved with a bioabsorbable polymer so as to form a sheet like bioabsorbable polymer material; cylindrically rolling the sheet like bioabsorbable polymer material; non-freeze-drying a solvent of a solution dissolved with a bioabsorbable polymer so as to form a sheet like bioabsorbable polymer material; cylindrically wrapping the non-freeze-dried sheet-like bioabsorbable polymer material around the freeze-dried sheet-like bioabsorbable polymer material so as to from an overlapped sheet part; and heat sealing or solvent meld sealing at least a portion around an end edge on the outer peripheral side of the overlapped sheet part.

## Description

The present invention relates to bioabsorbable tube for regenerating nerve tissue, and a production method thereof.

As a main treatment method for damaged nerve tissue, a treatment method in which an artificial tube is used so as to keep a space for regenerating nerve tissue has been tried. As a result of this, it has been found out that nerve tissue can be re-joined even if it is cut so as not to be unjoinable by an operation.

As for the artificial tube, an artificial tube made of a synthetic polymer material such as silicone, nitrocellulose or the like has been tried. However, as for the artificial tube made of such the material, since the permeability of a material being necessary for growing nerve cells is low, there are problems that the regeneration of nerve tissue is prevented and the tube is remained in a body as a foreign substance after the nerve tissue is regenerated. Thus, when the re-operation for removing the remained artificial tube is carried out, the regenerated nerve tissue may be damaged.

Therefore, the following materials have been proposed, that is, a neuroregeneration assisting material consisting of a bundle of collagen fibers coated with laminine and fibronectine (for example, refer to Japanese Patent Application Laid Open No. 5-237139), and a base material for neuranagenesis in which fibers including a material with absorptivity in an organism are bundled, where the material is a synthetic polymer such as polylactic acid, polyglycolic acid or the like, and a natural polymer such as collagen, chitin, chitosan, hyaluronic acid or the like (for example, refer to Japanese Patent Application Laid Open No. 2000-325463). As for an artificial tube consisting of such the bioabsorbable polymer material, since a constituent material is a bundle of fibers, material permeability resulting from spaces remained betweenfibersisexcellent. Further, since this artificial tube is finally absorbed in an organism, removal by the re-operation is not necessary. Further, this artificial tube has flexibility so that peripheral tissue is not damaged. Thus, this artificial tube has many advantages. However, since the constitution material is a bundle of fibers, there is a problem that a production method of a cylindrical artificial tube is complicated. Further, collagen is a material originating from an organism, so that there is a problem in a safety with respect to an unknown pathogen when the collagen is used as a medical material.

Further, a tube for neuroregeneration in which a fiber bundle consisting of a synthetic bioabsorbable polymer material is contained in the lumen of a cylindrical body including a bioabsorbable polymer material so as to have proper strength and flexibility has been indicated (for example, refer to Japanese Patent Application Laid Open No. 2005-143979). The tube for neuroregeneration is produced by mounting a fiber consisting of a synthetic bioabsorbable polymer material on a rod made of metal, dipping it in a solution of a synthetic bioabsorbable polymer material, freeze-drying it, removing the rod made of metal, and reversing the fiber. The tube for neuroregeneration has an inner layer formed with a synthetic bioabsorbable polymer sponge, and an outer layer formed with a synthetic bioabsorbable polymer fiber. However, this tube needs a complicated process for freeze-drying the fiber, removing the rod made of metal, and reversing the fiber. Thus, there is a problem of low productivity.

Further, a tube for neuroregeneration consisting of a synthetic bioabsorbable polymer material having comparatively high strength has been indicated (for example, refer to Japanese Patent Application Laid Open No. 2003-19196). The tube for neuroregeneration includes a sponge consisting of a synthetic bioabsorbable polymer material and a cylindrical reinforcing material consisting of a synthetic bioabsorbable polymer material which has a longer decomposition and absorption period than the sponge. Further, in this tube, at least an inner face is formed with a sponge. A production method of this tube for neuroregeneration includes the following processes (A) to (C) .
Process (A) : Fixing a cylindrical reinforcing material consisting of a synthetic bioabsorbable fiber on the outside of a cylindrical core body.
Process (B) : Dipping the obtained core body fixed with the reinforcing material in a synthetic bioabsorbable polymer solution, freeze-drying it, and thereby forming a sponge layer having a shorter decomposition and absorption period than the cylindrical reinforcing material.
Process (C) : Removing the freeze-dried material from the cylindrical core body, and reversing the fiber if necessary.

In the production method of the tube for neuroregeneration, when the tube having a sponge-like bioabsorbable material on the inner face is produced, the tube produced by the above-described method is necessary to be reversed, so that there are problems that the production method is complicated and thus productivity is low. Further, in the same patent document, the followings were described as the other production method. That is, "the cylindrical reinforcing material can be fixed at a position distant from the cylindrical core body by providing a detachable projection part (for example, a radiate projection, a donut shaped flange or the like) for fixing the cylindrical reinforcing material at a specified position of the cylindrical core body (for example, a position corresponding to the length of the tube for neuroregeneration). In this case, the reinforcing material or the projection part has an opening so as to invade a synthetic bioabsorbable polymer solution into a space between the reinforcing material and the cylindrical core body. By fixing the cylindrical reinforcing material at the position distant from the cylindrical core body, the tube for neuroregeneration in which the reinforcing material is integrated with the sponge (the reinforcing material is surrounded by the sponge) can be obtained." However, since this method also needs a specific core material, there is a problem in remarkably low production efficiency.

As described above, as for the conventional tube consisting of a synthetic bioabsorbable polymer material for regenerating nerve tissue, the production method is complicated, and the production cost is high. Thus, many kinds of tubes having different thickness, inner diameters and outer shapes are hardly formed, so that there is a problem that products corresponding to various kinds and modes of nerve portions cannot rapidly provided to a medical site.

An obj ective of the present invention is to provide a bioabsorbable tube for regenerating nerve tissue and a production method thereof, where the tube can be produced easier than a conventional bioabsorbable tube for regenerating nerve tissue with a low cost, and therefore, various kinds of the tubes having different thicknesses, inner diameters and outer shapes can be easily formed.

The earnest work was carried out in order to solve the above-mentioned problems and, as a result of this, present inventors found out the followings to complete the present invention. That is, a sheet-like bioabsorbable polymer material is produced at first by not using a bundle of synthetic bioabsorbable fibers, but thinly spreading and drying a solution dissolved with a bioabsorbable polymer so as to remove a solvent. Then, a cylindrical tube of the sheet-like bioabsorbable polymer material having an overlapped part is formed by rolling it, and at least a portion around an end edge on the outer peripheral side of the overlapping sheet part is heat sealed or solvent meld sealed. As a result, by only changing the thickness of the sheet, the combination of sheet materials, the rolling diameter and the number of rolling times, various kinds and modes of tubes for regenerating nerve tissue can be easily produced.

That is, the present invention is a bioabsorbable tube which is produced by: drying a solvent of a solution dissolved with a bioabsorbable polymer so as to form a sheet-like bioabsorbable polymer material; cylindrically rolling the sheet-like bioabsorbable polymer material so as to form an overlapped sheet part; and heat sealing or solvent meld sealing at least a portion around an end edge on the outer peripheral side of the overlapped sheet part.

Further, the present invention is a production method of a bioabsorbable tube, the method comprising: drying a solvent of a solution dissolved with a bioabsorbable polymer as to form a sheet-like bioabsorbable polymer material; cylindrically rolling the sheet-like bioabsorbable polymer material so as to form an overlapped sheet part; and heat sealing or solvent meld sealing at least a portion around an end edge on the outer peripheral side of the overlapped sheet part so as to form a tubular shape.

Further, the present invention is a bioabsorbable tube having a two layers structure, the tube produced by: freeze-drying a solvent of a solution dissolved with a bioabsorbable polymer so as to form a sheet-like bioabsorbable polymer material; cylindrically rolling the sheet-like bioabsorbable polymer material; non-freeze-drying a solvent of a solution dissolved with a bioabsorbable polymer so as to form a cylindrical sheet-like bioabsorbable polymer material; cylindrically wrapping the non-freeze-dried sheet-like bioabsorbable polymer material around the cylindrical freeze-dried sheet-like bioabsorbable polymer material so as to from an overlapped sheet part; and heat sealing or solvent meld sealing at least a portion around an end edge on the outer peripheral side of the overlapped sheet part.

Further, the present invention is a production method of a bioabsorbable tube having a two layers structure, the method comprising: freeze-drying a solvent of a solution dissolved with a bioabsorbable polymer so as to forma sheet-like bioabsorbable polymer material; cylindrically rolling the sheet-like bioabsorbable polymer material; non-freeze-drying a solvent of a solution dissolved with a bioabsorbable polymer so as to form a cylindrical sheet-like bioabsorbable polymer material ; cylindrically wrapping the non-freeze-dried sheet-like bioabsorbable polymer material around the cylindrical freeze-dried sheet-like bioabsorbable polymer material so as to from an overlapped sheet part; and heat sealing or solvent meld sealing at least a portion around an end edge on the outer peripheral side of the overlapped sheet part .

In the above-described first bioabsorbable tube and the production method of this bioabsorbable tube according to the present invention, the thickness of the sheet-like bioabsorbable polymer material is preferably 0.01 to 2 mm. At this time, when a drying method of the solvent is a freeze-drying method, a sponge-like sheet can be produced, and when a drying method of the solvent is a non-freeze-drying method, a bioabsorbable tube having excellent breakage resistance can be produced.

Further, in the above-described second bioabsorbable tube having a two layers structure and the production method of this bioabsorbable tube according to the present invention, the sponge and sheet like bioabsorbable polymer material, which is formed by freeze-drying the solution dissolved with the bioabsorbable polymer, is cylindrically rolled as an inner layer, and the sheet-like bioabsorbable polymer material having excellent breakage resistance, which is formed by non-freeze-drying the solution dissolve with the bioabsorbable polymer, is cylindrically wrapped around the inner layer as an outer layer, so as to form the overlapped sheet part. Then, at least the portion around an end edge on the outer peripheral side of the overlapped sheet part is heat sealed or solvent meld sealed. Thus, since the side of the tube facing nerve tissue to be regenerated is in the sponge state, the nerve tissue to be regenerated is not pressed, and body fluid and blood can be kept in the tube. As a result, the bioabsorbable tube produced by this method is preferable for regenerating nerve tissue, and since the outer face of the tube has excellent breakage resistance, there are no problems that the regenerated nerve tissue is broken during the regenerating process and a hollow part is closed.

Further, in such the bioabsorbable tube and the production method of a bioabsorbable tube according to the present invention, at least one or more kinds of synthetic high polymers selected from the followings can be preferably used as the bioabsorbable polymer. That is, polyglycolic acid, polylactic acid (D body, L body, DL body), poly-ε- caprolactone, poly-P-dioxanone, a lactic acid-ε-caprolactone copolymer, a lactic acid-glycolic acid copolymer, a glycolic acid-trimethylene carbonate copolymer, a glycolic acid-trimethylene carbonate-P-dioxanone copolymer, and a glycolic acid-trimethylene carbonate-ε-caprolactone copolymer.

The bioabsorbable tube according to the present invention is produced by: producing a bioabsorbable polymer material sheet by thinly spreading and drying a solution dissolved with a bioabsorbable polymer so as to remove a solvent; cylindrically rolling the sheet so as to form an overlapped part; and heat sealing or solvent meld sealing at least a portion around an end edge on the outer peripheral side of the overlapped part of the cylindrical sheet. So, by only changing the thickness of the sheet, the combination of sheet materials, the rolling diameter and the number of rolling or wrapping times, various kinds and modes of bioabsorbable tubes can be easily produced. Further, the production method of an bioabsorbable tube according to the present invention can produce various kinds and modes of bioabsorbable tubes easier than the conventional production methods of a bioabsorbable tube using a reinforcing material such as a synthetic bioabsorbable fiber or the like. So, a bioabsorbable tube suitable to various kinds of medical treatments of a portion in which nerve tissue is damaged can be provided, so that the present invention has a remarkable value to contribute to a medical field.

In order to produce a bioabsorbable tube according to the present invention, a sheet-like bioabsorbable polymer material is produced by thinly spreading a solution dissolved with a bioabsorbable polymer, and drying it so as to remove a solvent at first.

As the bioabsorbable polymer, a conventionally used bioabsorbable polymer material can be used. More particularly, polyglycolic acid, polylactic acid (D body, L body, DL body), poly-ε- caprolactone, poly-P-dioxanone or a copolymer of them can be preferably used as aliphatic polyester. More particularly, the copolymers are a lactic acid-ε-caprolactone copolymer, a lactic acid-glycolic acid copolymer, aglycolicacid-trimethylene carbonate copolymer, a glycolic acid-trimethylene carbonate-P-dioxanone copolymer, and a glycolic acid-trimethylene carbonate-ε-caprolactone copolymer. Further, a copolymer of the aliphatic polyester and polyester ether can be used, and one or more kinds of these bioabsorbable polymers can be used.

In these copolymers, when at least one or more kinds of combined copolymers selected from polyglycolic acid, polylactic acid (D body, L body, DL body), poly-ε-caprolactone, a lactic acid-ε-caprolactone copolymer, and a lactic acid-glycolic acid copolymer are used, it is preferable that excellent breakage resistance can be obtained when a bioabsorbable tube according to the present invention is produced by non-freeze-drying.

A solution dissolved with such the bioabsorbable polymer materials is uniformly spread so as to have the desired thickness and dried so as to produce a sheet-like bioabsorbable polymer material. The thickness is preferably 0.01 to 2 mm. If the thickness is less than 0.01 mm, the sheet having the uniform thickness is hardly obtained. If the thickness is more than 2 mm, the tube is not preferable as a conventional tube for regenerating nerve tissue. In addition, a solvent can be suitably selected corresponding to the polymer material to be used. For example, chloroform, dichloromethane, carbon tetrachloride, acetone, dioxane, and tetrahydrofuran can be used.

As for the drying method of the solvent, the conventional freeze-drying is used. The bioabsorbable polymer material having a sponge-like structure can be produced by freeze-drying. Further, the bioabsorbable polymer material may be produced by a non-freeze-drying such as an ordinary temperature drying or a combination of the ordinary temperature drying and a vacuum drying. By using such the method, the breakage resistance of the bioabsorbable polymer material can be increased.

The produced sheet-like bioabsorbable polymer material is formed so as to have a tubular state by rolling and overlapping a sheet part, and at least a portion around an end edge on the outer peripheral side of the overlapped sheet part is heat sealed or solvent meld sealed so as to keep the tubular shape. As a method for heat sealing or solvent meld sealing at least a portion around the end edge on the outer peripheral side of the overlapped sheet part, for example, the following methods can be used, that is, a method for heating the polymer of the overlapped sheet part at a melting point or more so as to seal the sheet part, and a method for applying a solvent to the overlapped sheet part so as to dissolve the polymer, and thereby sealing the sheet part. When the bioabsorbable polymer material sheet is rolled, a core material such as a rod or the like can be used, but the core material cannot be used depending on the conditions of the thickness of the sheet or the like. Further, the number of rolling or wrapping times can be freely selected depending on the characteristic of the bioabsorbable polymer material sheet, the needed application of the completed bioabsorbable tube, and a portion in a living body where the tube is used.

Further, the bioabsorbable tube having a two layers structure can be easily produced by: rolling the bioabsorbable polymer material sheet having a sponge-like structure, which is produced by freeze-drying, so as to have a tube shape; and rolling the bioabsorbable polymer sheet not having a sponge-like structure, which is produced by non-freeze-drying, on the outer side of the freeze-dried bioabsorbable polymer material sheet. By this method, the combination, shape and size of desired materials can be easily adjusted to be produced. As for the bioabsorbable tube having a two layers structure, since the inner layer has a sponge-like structure, nerve tissue to be regenerated is not pressed, and body fluid and blood can be kept in the tube, so that it is preferable to regenerate nerve tissue. Further, since the outer layer has excellent breakage resistance, there are no problems that the regenerated nerve tissue is broken during the regenerating process and a hollow part is closed. Thus, the bioabsorbable tube is excellent.

### [Example]

### <Production of a bioabsorbable polymer material sheet>

As shown in Table 1, a bioabsorbable polymer material sheet was produced by: producing a solution by dissolving a bioabsorbable polymer in a solvent; taking it into a glass mold having the size of 100 mm×100 mmx20 mm to have an objective thickness; and drying it to remove the solvent.

**[Table 1]**

| | Sheet 1 | Sheet 2 | Sheet 3 | Sheet 4 |
|---|---|---|---|---|
| Solvent | 1,4 dioxane | Dichloromethane | Dichloromethane | 1,4 dioxane |
| Bioabsorbable polymer, | Lactic acid-slycolic acid copolymer (Lactic acid: Glycolic acid=75:25), | (Poly-(L)-lactic | Lactic acid-ε-caprolactone copolymer (Lactic acid: ε-caprolactone=80:20) | Lactic acid-glycolic acid copolyme (Lactic acid:Glycolic acid=75:25) |
| Weight average molecular weight | About 250,000 | About 200,000 | About 500,000 | About 250,000 |
| Concentration (weight %) | 8 | 6 | 5 | 8 |
| Drying method | Freeze-drying | Freeze-drying | Non- freeze-drying | Non-freeze-drying |
| Average size of pore (µm) | 25 | 25 | - | - |
| Final thickness (µm) | 250 | 250 | 40 | 40 |

| | | | | |
|---|---|---|---|---|
| Freeze-drying: The solvent was removed by freezing at -30 degree C for 2 hours using a freezer (the product name: MDF-0281AT, produced by Sanyo Electric Corporation), and drying for 48 hours under decompression using a vacuum dryer (the product name: DP43, produced by Yamato Chemical Corporation). Non-freeze-drying: The solvent was removed by drying for 48 hours at a room temperature (23 degree C), and drying for 48 hours under decompression using a vacuum dryer (the product name: DP43, produced by Yamato Chemical Corporation). | | | | |

### <Example 1>

A bioabsorbable tube having the inner diameter of about 2 mm and the outer diameter of about 2.5 mm was produced by: cutting a sheet 1 to have the size of 50 mmx6.5 mm; wrapping the sheet one time around a core material having a fluorine coating film from the long side and having the diameter of 2 mm (the overlapped part was about 0.5 mm) on a hot plate at 40 degree C; heat sealing an end part of the overlapped part on a hot plate at 80 degree C; and removing the core material.

### <Example 2>

A bioabsorbable tube having the inner diameter of about 2 mm and the outer diameter of about 3 mm was produced by: cutting a sheet 2 to have the size of 50 mm×14 mm; wrapping the sheet two times around a core material having a fluorine coating film from the long side and having the diameter of 2 mm on a hot plate at 60 degree C; allowing standing it for 3 minutes in a dryer at 180 degree C so as to seal an overlapped part; and removing the core material.

### <Example 3>

A bioabsorbable tube having a two layers structure, in which the inner diameter was about 1 mm and the outer diameter was about 1.7 mm, was produced by: cutting a sheet 1 to have the size of 50 mmx3.5 mm; wrapping the sheet one time around a core material having a fluorine coating film from the long side and having the diameter of 1 mm (the overlapped part was about 0.5 mm) on a hot plate at 40 degree C; heat sealing an end part of the overlapped part on a hot plate at 80 degree C; wrapping a sheet 4 cut to have the size of 50 mm×15 mm three times around the outer side of the sheet 1 from the long side; allowing standing it for 3 minutes in a dryer at 80 degree C so as to seal an overlapped part; and removing the core material.

### <Example 4>

A bioabsorbable tube having a two layers structure, in which the inner diameter was about 2 mm and the outer diameter was about 3.3 mm, was produced by: cutting a sheet 1 to have the size of 50 mm×14 mm; wrapping the sheet two times around a core material having a fluorine coating film from the long side and having the diameter of 2 mm on a hot plate at 80 degree C; heat sealing an end part of the overlapped part on a hot plate at 80 degree C; wrapping a sheet 4 cut to have the size of 50 mmx39 mm four times around the outer side of the sheet 1 from the long side; allowing standing it for 3 minutes in a dryer at 80 degree C so as to seal an overlapped part ; and removing the core material .

### <Example 5>

A bioabsorbable tube having a two layers structure, in which the inner diameter was about 2 mm and the outer diameter was about 2.8 mm, was produced by: cutting a sheet 2 to have the size of 50 mmx6.5 mm; wrapping the sheet about one time around a core material having a fluorine coating film from the long side and having the diameter of 2 mm (the overlapped part was about 0.5 mm) on a hot plate at 60 degree C; applying 1, 4 dioxane on a portion apart about 0.5 mm from an end of a completed overlapped part so as to seal the overlapped part; wrapping a sheet 3 cut to have the size of 50 mmx33 mm four times around the outer side of the sheet 2 from the long side; applying 1, 4 dioxane on a portion apart about 1 mm from an end of a completed overlapped part so as to seal the overlapped part; allowing standing it for 3 minutes in a dryer at 80 degree C; and removing the core material.

### <Example 6>

A bioabsorbable tube having the inner diameter of about 2 mm and the outer diameter of about 2.6 mm was produced by: cutting a sheet 3 to have the size of 50 mm×57 mm; wrapping it about eight times around a core material having a fluorine coating film from the short side and having the diameter of 2 mm; applying dichloromethane to a portion apart about 1 mm from an end of a completed overlapped part so as to solvent meld seal the overlapped part; and removing the core material.

### <Example 7>

A bioabsorbable tube having the inner diameter of about 3 mm and the outer diameter of about 3.8 mm was produced by: cutting a sheet 3 to have the size of 50 mm×106 mm; wrapping it about ten times around a core material having a fluorine coating film from the short side and having the diameter of 3 mm; allowing standing it in a dryer at 140 degree C for 3 minutes so as to seal the overlapped part; and removing the core material.

### <Example 8>

A bioabsorbable tube having the inner diameter of about 2 mm and the outer diameter of about 3.2 mm was produced by: cutting a sheet 4 to have the size of 50 mm×121 mm; wrapping it about five times around a core material having a fluorine coating film from the short side and having the diameter of 2 mm; allowing standing it in a dryer at 80 degree C for 3 minutes so as to seal the overlapped part; and removing the core material.

### <Example 9>

A bioabsorbable tube having the inner diameter of about 3 mm and the outer diameter of about 3.8 mm was produced by: cutting a sheet 4 to have the size of 50 mm×106 mm; wrapping it about ten times around a core material having a fluorine coating film from the short side and having the diameter of 3 mm; allowing standing it in a dryer at 80 degree C for 3 minutes so as to seal the overlapped part; and removing the core material.

### <Example 10>

A bioabsorbable tube having the inner diameter of about 4 mm and the outer diameter of about 4.4 mm was produced by: cutting a sheet 4 to have the size of 50 mm×65 mm; wrapping it about five times around a core material having a fluorine coating film from the short side and having the diameter of 4 mm; sealing about 1 mm of the end part of an overlapped part on a hot plate at 80 degree C; and removing the core material.

### <Measuring of curvature (the test of breakage resistance)>

Each bioabsorbable tube was cut to have the length of 3 cm. While contacting near the center of each bioabsorbable tube to a pipe having the diameter of 0.95 cm at 37 degree C, a force was gradually applied from both ends of the bioabsorbable tube so as to curve the whole of the bioabsorbable tube to have a U shape. When each bioabsorbable tube could be curved while keeping a hollow shape, the distance (Wcm) between the both ends of the tube was measured. The measured distance was applied to the formula "(1-W/3)×100" so as to calculate the curvature (%). Results were collectively shown in Table 2. In addition, in Table 2, "Bend" showed that the bioabsorbable tube could not keep the hollow shape.

**[Table 2]**

| | Used sheet | | Inner diameter (mm) | Outer diameter (mm) | Curvature | State |
|---|---|---|---|---|---|---|
| | Inner side | Outer side | | | | |
| Example 1 | 1 | - | 2.0 | 2.5 | 16% | Bend |
| Example 2 | 2 | - | 2.0 | 3.0 | 21% | Bend |
| Example 3 | 1 | 4 | 1.0 | 1.7 | 100% | Wrapped around a pipe without Bend |
| Example 4 | 1 | 4 | 2.0 | 3.3 | 100% | Wrapped around a pipe without Bend |
| Example 5 | 2 | 3 | 2.0 | 2.8 | 100% | Wrapped around a pipe without Bend |
| Example 6 | 3 | - | 2.0 | 2.6 | 100% | Wrapped around a pipe without Bend |
| Example 7 | 3 | - | 3.0 | 3.8 | 100% | Wrapped around a pipe without Bend |
| Example 8 | 4 | - | 2.0 | 3.2 | 100% | Wrapped around a pipe without Bend |
| Example 9 | 4 | - | 3.0 | 3.8 | 100% | Wrapped around a pipe without Bend |
| Example 10 | 4 | - | 4.0 | 4.4 | 100% | Wrapped around a pipe without Bend |

Clearly from Table 2, the production method of a bioabsorbable tube according to the present invention can easily produce various kinds and modes of bioabsorbable tubes by only changing the thickness of a sheet, the combination of sheet materials, the rolling diameter, the rolling or wrapping times and the like.

Further, the followings were confirmed. That is, the bioabsorbable tube was produced using the bioabsorbable polymer material sheet having a sponge-like structure produced by freeze-drying so as to remove the solvent. This tube was bent when it was curved to have a small diameter. However, it was confirmed that when the bioabsorbable tube was produced using the bioabsorbable polymer material sheet produced by non-freeze-drying so as to remove the solvent, the tube was not bent.

Further, the bioabsorbable tube having a two layers structure was produced by rolling the bioabsorbable polymer material sheet produced by non-freeze-drying so as to remove the solvent, on the outer side of the bioabsorbable polymer material sheet having a sponge-like structure. The sponge-like structure was produced by freeze-drying so as to remove the solvent. As for this tube having a two layers structure, although the bioabsorbable polymer material having the sponge-like structure, which was the inner material, was cracked, the tube was not bent.

## Claims

1. A bioabsorbable tube comprising: drying a solvent of a solution dissolved with a bioabsorbable polymer so as to form a sheet-like bioabsorbable polymer material;
cylindrically rolling the sheet-like bioabsorbable polymer material so as to form an overlapped sheet part ; and
heat sealing or solvent meld sealing at least a portion around an end edge on the outer peripheral side of the overlapped sheet part.

2. The bioabsorbable tube according to claim 1, wherein the thickness of the sheet-like bioabsorbable polymer material is 0.01 to 2 mm.

3. The bioabsorbable tube according to claim 1 or 2, wherein the sheet like bioabsorbable polymer material is produced by non-freeze-drying a solvent.

4. The bioabsorbable tube according to claim 1 or 2, wherein the sheet like bioabsorbable polymer material is produced by freeze-drying a solvent.

5. A bioabsorbable tube having a two layers structure, produced by:
freeze-drying a solvent of a solution dissolved with a bioabsorbable polymer so as to form a sheet-like bioabsorbable polymer material; cylindrically rolling the sheet-like bioabsorbable polymer material;
non-freeze-drying a solvent of a solution dissolved with a bioabsorbable polymer so as to form a sheet-like bioabsorbable polymer material;
cylindrically wrapping the non-freeze-dried sheet-like bioabsorbable polymer material around the freeze-dried sheet-like bioabsorbable polymer material so as to form an overlapped sheet part; and
heat sealing or solvent meld sealing at least a portion around an end edge on the outer peripheral side of the overlapped sheet part.

6. The bioabsorbable tube according to claim 3 or 5, wherein the tube has breakage resistance in which the tube is not bent when the tube having the length of 3 cm is wound on a pipe having the diameter of 0.95 cm at 37 degree C.

7. A bioabsorbable tube according to any one claims 1 to 6,
wherein the bioabsorbable polymer is at least one or more kinds of synthetic polymers selected from polyglycolic acid, polylactic acid (D body, L body, DL body), poly-ε- caprolactone, poly-P-dioxanone, a lactic acid-ε-caprolactone copolymer,a lactic acid-glycolic acid copolymer, a glycolic acid-trimethylene carbonate copolymer, a glycolic acid-trimethylene carbonate-P-dioxanone copolymer, and a glycolic acid-trimethylene carbonate-ε-caprolactone copolymer.

8. A production method of a bioabsorbable tube, the method comprising:
drying a solvent of a solution dissolved with a bioabsorbable polymer as to form a sheet-like bioabsorbable polymer material;
cylindrically rolling the sheet-like bioabsorbable polymer material so as to form an overlapped sheet part; and heat sealing or solvent meld sealing at least a portion around an end edge on the outer peripheral side of the overlapped sheet part so as to form a tubular shape.

9. The production method of a bioabsorbable tube according to claim 8,
wherein the thickness of the sheet-like bioabsorbable polymer material is 0.01 to 2 mm.

10. The production method of a bioabsorbable tube according to claim 8 or 9,
wherein a drying method for the solvent is freeze-drying.

11. The production method of a bioabsorbable tube according to claim 8 or 9,
wherein a drying method for the solvent is non-freeze-drying.

12. A production method of a bioabsorbable tube having a two layers structure, the method copmrising:
freeze-drying a solvent of a solution dissolved with a bioabsorbable polymer so as to form a sheet-like bioabsorbable polymer material;
cylindrically rolling the sheet-like bioabsorbable polymer material;
non-freeze-drying a solvent of a solution dissolved with a bioabsorbable polymer so as to form a sheet-like bioabsorbable polymer material;
cylindrically wrapping the non-freeze-dried sheet-like bioabsorbable polymer material around the freeze-dried sheet-like bioabsorbable polymer material so as to form an overlapped sheet part;
and heat sealing or solvent meld sealing at least a portion around an end edge on the outer peripheral side of the overlapped sheet part.

13. A production method of a bioabsorbable tube having a two layers structure according to any one claims 8 to 12,
wherein the bioabsorbable polymer is at least one or more kinds of synthetic high polymers selected from polyglycolic acid, polylactic acid (D body, L body, DL body), poly-ε- caprolactone, poly-P-dioxanone, a lactic acid-ε-caprolactone copolymer, a lactic acid-glycolic acid copolymer, a glycolic acid-trimethylene carbonate copolymer, a glycolic acid-trimethylene carbonate-P-dioxanone copolymer, and a glycolic acid-trimethylene carbonate-ε-caprolactone copolymer.
